Europäisches Patentamt
European Patent Office
Office européen des brevets

(11) Publication number: 0 204 527 A1

# EUROPEAN PATENT APPLICATION

(21) Application number: 86304144.8

(22) Date of filing: 30.05.86

(51) Int. Cl.[4]: C12P 21/00 , C12P 21/02 , C12P 21/06 , C12N 15/00

The applicant has filed a statement in accordance with Rule 28 (4) EPC (issue of samples only to experts).

(30) Priority: 04.06.85 PCT/JP85/00308

(43) Date of publication of application: 10.12.86 Bulletin 86/50

(84) Designated Contracting States: AT BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Nakagawa, Shizue
23-709, 6 Nankounaka 5-chome Suminoe-Ku
Osaka-Shi Osaka 559(JP)
Inventor: Yamada, Takao
552, Hitotsuya-Cho
Matsubara-Shi Osaka 580(JP)
Inventor: Nishimura, Osamu
54-16, Daiwanishi 1-chome
Kawanishi-Shi Hyogo 666-01(JP)

(74) Representative: Lewin, John Harvey et al
Elkington and Fife High Holborn House 52/54
High Holborn
London WC1V 6SH(GB)

## (54) Removal of the N-terminal methionine from methionylproteins.

(57) The N-terminal methionine in the methionyl-protein can be efficiently removed by reacting methionylprotein with aminopeptidase.

EP 0 204 527 A1

# REMOVAL OF THE N-TERMINAL METHIONINE FROM METHIONYLPROTEINS

The present invention relates to a method for removal of the N-terminal methionine from methionylproteins.

When a protein is biosynthesized within a cell, its amino terminus is always occupied by methionine (formylmethionine in case of procaryotes), which corresponds to the mRNA initiation codon, i.e. AUG. In produced mature protein molecules, however, such methionine is usually no longer present, because it is removed via subsequent processing.

On the other hand, recent rapid progress in gene recombination technology has made it possible to produce useful proteins using microbes or animal cells, e.g. Escherichia coli. In some cases the protein produced retains the methionine described above. For example, in non-glycosylated human interleukin-2 possessing the amino acid sequence shown in Fig. 1 (rIL-2) expressed by Escherichia coli, in addition to the molecule type whose initiation amino acid is alanine, as in the case of natural human interleukin-2, another type of molecule with methionine added at the amino terminus (Met-rIL-2) was found. Moreover, it was revealed that the methionine addition rate reaches 50% in interferon-α [Journal of Interferon Research, 1, 381 (1981)] or approx. 100% in human growth hormone [Nature, 293, 408 (1981)].

It can be conjectured that three dimensional structure, bioactivity and stability can differ between a molecule with methionine at its amino terminus and one without methionine, even though they be molecules of the same protein; addition of methionine at the amino terminus is thought of possibly causing an increase in protein antigenicity. Therefore, it would be significant, in terms of industrial application, to establish a method of removing such amino terminal methionine.

In order to dissolve this problem, a process was suggested by which methionine could be removed by cyanogen bromide (BrCN) cleavage - [Science, 198, 1056 (1977)] ; however, no satisfactory result has been obtained, since the process not only premises the absence of other methionine residue(s) in the molecule of the required mature protein but also subjects the protein to a drastic chemical reaction.

PCT International Publication No. W084/02351 discloses a method for producing a mature protein in which fused protein having a pre-sequence or codon sequence composed of two or more amino acids is decomposed by aminopeptidase; however, this method cannot cleave amino terminal methionine alone and thus cannot achieve the purpose stated above.

The present inventors, after studying how to produce polypeptide possessing an amino acid sequence equaling that of natural polypeptide by cleaving amino terminal methionine alone from polypeptide synthesized by gene engineering techniques, found that amino terminal methionine alone can be cleaved specifically from some kinds of polypeptides by causing a reaction, using aminopeptidase, to those polypeptides. The finding was followed by further research, leading to the completion of the invention.

The present invention is concerned with a method for removal of the N-terminal methionine from a methionylprotein represented by the formula:

H-Met-X-Pro-Y-OH (I)

wherein X is an amino acid other than proline, Y is a peptide chain, which comprises reacting the methionylprotein (I) with aminopeptidase to give a protein represented by the formula:

H-X-Pro-Y-OH (II)

wherein X and Y have the same meanings as defined above.

Amino acids which is defined by X in the formula (I) include all amino acids other than Pro. The examples of the amino acids are essential amino acids, e.g. Ala, Phe, Arg, Thr, Ile, Gln, Ser, Gly and Leu. Ala, Phe, Arg and Thr are especially preferable among others.

Peptide chains which are defined by Y include any peptides composed of 2 or more amino acids and possessing an arbitrary amino acid number, preferably less than about 400. Preferable molecular weight for the said peptide chain is about 200 to 50,000.

From the practical point of view, the use of peptide chain of about 20 to 200 amino acid number or of about 200 to 36,000 molecular weight is recommended; such peptide chain include those corresponding to Y in polypeptide produced via gene engineering techniques or peptide produced via chemical synthesis.

Polypeptides represented by the formula (II), produced by the present method, include, for example, the following: lymphokines such as human interleukin-2 (human IL-2); hormones such as growth hormone, e.g. human growth hormone, horse growth hormone, bovine prolactin, bovine pancreatic hormone and chicken pancreatic hormone; enzyme inhibitors such as bovine basic pancreatic trypsin inhibitor and dog submandibular

protease inhibitor; substance P; blood proteins such as human blood plasma albumin; Desulfovibrio vulgaris flavotoxin; pig cardiac muscular aspartate aminotransferase; rabbit skeletal muscular triosephosphate isomerase; papaya papain; Streptococcus pyogenes protease; Myxobacter 495β lytic protease; cholera toxin β chain; bovine kallidin I, Rana japonica kallidin I and human kallidin I; human serumal very low density lipoprotein C-I; cow's milk αS, casein B variant; Escherichia coli L-asparaginase; etc. Known or novel polypeptides expressed by the formula (II) and produced via chemical synthesis etc. are also exemplified.

In the present specification, polypeptides include all peptides and proteins composed of two or more amino acids, whether glycosylated or nongylcosylated.

Among Polypeptide (II), human interleukin-2 and human growth hormone are preferable.

Said human interleukin-2 includes all substances possessing biological or immunological activities similar to those of natural human interleukin-2, e.g. binding activity with interleukin-2 receptor or anti-interleukin-2 antibody; specifically, polypeptides possessing an amino acid sequence as shown in Fig. 1; their fragments composed of a partial amino acid sequence essential to their biological or immunological activity are included, e.g. a fragment lacking one amino acid at the amino terminus (European Patent Application Publication No. 91539), a fragment lacking four amino acids at the amino terminus (Japanese Patent Application Laid-open No. 126088/1985), fragments lacking several amino acids at the carboxyl terminus. Also included are polypeptides possessing an amino acid sequence as shown in Fig. 1 in which some amino acids are lost or replaced by other amino acids, e.g. those in which the No.125 cysteine residue is replaced by a serine residue (U.S. Patent No. 4,518,584).

Aminopeptidases which can be used include known peptidases, e.g. aminopeptidases M [EC 3.4.11.2; Methods in Enzymology, 19, 514 (1971)], leucine aminopeptidases [EC 3.4.11.1; The Enzymes, 3, 81 (1971)], aminopeptidase PO [Protein, Nucleic Acid and Enzyme, 28, 1220 (1983) published by Kyoritsu Shuppan Co. Ltd., Japan], aminopeptidase B [EC 3.4.11.6; The Enzymes, 3, 112 (1971)], and aminopeptidase P [EC 3.4.11.9; Biochemical Biological Research Communications, 32, 658 (1968)]; these aminopeptidases are available from suppliers and can be produced by the methods described in said papers.

Among the aminopeptidases, amionpeptidase M is preferable.

When human IL-2 in the form of Polypeptide (II) is to be produced by the present method, using as a starting material human IL-2 possessing an H-Met-Ala-Pro fragment at its amino terminus in the form of Polypeptide (I), the use of aminopeptidase M as an aminopeptidase is especially preferable.

In the present invention, Polypeptide (II) is produced by subjecting Polypeptide (I) to a reaction with aminopeptidase.

Polypeptide (I) to be used as a starting material includes both purified products and partially purified products obtained during the purification process; the concomitance of Polypeptide (II) is permitted.

It is recommended that Polypeptide (I) be a nonglycosylated polypeptide.

The use of a buffer solution in the reaction is recommended; buffer solutions which is used include all those composed of an inorganic base (e.g. sodium, potassium, ammonium) salt of an organic (e.g. acetic) or inorganic (e.g. phosphoric) acid and a solvent, as long as they do not inhibit said enzyme reaction.

Reaction pH is in the range of about 4 to 10; from the point of view of stability of both enzyme and polypeptide, a pH range of about 6 to 9 is especially preferable. Reaction time ranges from about 1 to 100 hours. Reaction temperature ranges from about 4 to 70°C, preferably between about 15°C and 70°C, more preferably between about 30°C and 50°C is recommended. The ratio of aminopeptidase to Polypeptide (I) as a substrate ranges from about 1/1,000 to 1/1 (molar ratio); from the point of view of yield, reaction time and cost, however, a molar ratio of about 1/100 to 1/5 is preferable. Where an available aminopeptidase is used which is mingled with dipeptidase, prolidase etc., it is recommended that the mingling enzymes be separated by ordinary methods such as chromatography or that their activity be inhibited by adding inhibitors such as diisopropyl fluorophosphate (DFP) and p-chloromercuribenzoic acid (PCMB), or that the aminopeptidase be treated by a combination of both methods.

In the present method, prior to use in the reaction, aminopeptidase may be immobilized with a carrier.

Carriers which are used in immobilization include any compounds which can combine with aminopeptidase; water-insoluble polymers possessing high affinity are especially favorable. Such polymers include homopolymers such as cellulose, its derivatives, crosslinked dextran, its derivatives, agarose, its derivatives, polyacrylamide, and copolymers such as agarose-polyacrylamide copolymer.

The covalent bond combination of aminopeptidase and a carrier may be achieved by combining them either directly or via a spacer. In direct combination, the carrier is activated beforehand so that it combines with the aminopeptidase; a method using a halogenated cyanogen such as cyanogen bromide is effective. In spacer-mediating combination, the spacer is incorporated into a carrier beforehand; aminopeptidase and spacer-incorporating carrier may be combined using water-soluble carbodiimides such as 1-ethyl-3-(3-dimethylaminopropyl)carbodiimide. Spacers used include compounds expressed by the formulas: $H_2N-(CH_2)_n-NH_2$ (n represents an integral number from 1 to 10.), $H_2N-(CH_2)_n-COOH$ (n represents and integral number from 1 to 10.), $H_2N-(CH_2)_nNH(CH_2)_m-NHCO-(CH_2)_n-COOH$(m represents an integral number from 1 to 3.), etc. Active ester derivatives obtained by esterifying the carboxyl group in said spacer with N-hydroxysuccinimide etc., is also used as spacers. Aminopeptidase and carrier are also combined by subjecting them to a reductive amination reaction using both a spacer incorporating an aldehyde group and sodium cyanoborohydride etc.

To combine a carrier and aminopeptidase via covalent bonds, an activated carrier or a carrier to which an active group has been incorporated and aminopeptidase are subjected to coupling reaction under the following conditions. These are mixed together at a pH of about 2 to 12 (preferably about 3 to 11) and at a temperature of about 0 to 50°C - (preferably about 4 to 30°C) and then stirred slowly for about 10 minutes to 24 hours (preferably about 2 to 16 hours); some salts (e.g. NaCl, $Na_2HPO_4$, etc.) which do not inhibit the reaction may be added to prevent the non-specific ionic adsorption of aminopeptidase to the carrier.

The resulting gel is collected via filtration or centrifugation. After the remaining active groups are blocked with primary amine such as glycine or ethylamine, the collected gel is rinsed well with phosphoric acid buffer solution etc. to yield a carrier bound with aminopeptidase, i.e. immobilized aminopeptidase.

Immobilized aminopeptidase can be used almost in the same manner as simple aminopeptidase; for example, Polypeptide (I), to be used as a starting material includes both purified and partially purified products obtained during the purification process. The use of a buffer solution is recommended; those which can be used include all composed of a sodium, potassium or ammonium salt of acids such as phosphoric, acetic and boric, as long as they do not inhibit the enzyme reaction. Reaction pH ranges from about 4 to 10; from the point of view of polypeptide stability, however, a pH range of about 6 to 9 is especially preferable. Reaction time ranges from about 1 to 200 hours, preferably from about 1 to 100 hours, reaction temperature from about 4 to 70°C, preferably from about 15 to 70°C, more preferably from about 30 to 50°C; it is recommended however that the reaction is carried out between about 4°C and 10°C, when the immobilized enzyme is repeatedly used.

Reaction progress can be monitored by analyzing some of the reactant liquid using methods based on the difference in isoelectric point, e.g. fast protein liquid chromatography (FPLC). Products can be identified by N-terminal sequencing or amino acid analysis.

Reaction products can be purified via the conventional methods of purifying protein or peptide: e.g. extraction, salting-out, distribution, recrystallization and chromatography, if required.

Polypeptide (II), produced by the present method, does not have Met at its amino terminus and possesses the same amino acid sequence as natural bioactive polypeptide. Therefore, it exhibits an activity equal to that of natural polypeptide and its toxicity is low; it can therefore be used safely as a drug and a diagnosis reagent.

The present invention provides a method for removal of the N-terminal methionine from methionylprotein in specifically.

When the aminopeptidase is employed as an immobilized form, the enzyme is used repeatedly, and therefore the method is in efficiency.

In the specification, claims and drawings of the present invention, amino acid abbreviations are based on those recommended by the IUPAC-IUB Commission on Biochemical Nomenclature, or those used commonly in the related field.

Examples are shown below (abbreviation represents L-type unless otherwise specified):

Gly : Glycine

Ala : Alanine

Val : Valine

Leu : Leucine

Ile : Isoleucine

Ser : Serine

Thr : Threonine

Cys : Cystein

Met : Methionine

Glu : Glutamic acid

Asp : Aspartic acid

Lys : Lysine

Arg : Arginine

His : Histidine

Phe : Phenylalanine

Tyr : Tyrosine

Trp : Tryptophan

Pro : Proline

Asn : Asparagine

Gln : Glutamine

Asp & Asn : Aspartic acid and asparagine

Glu & Gln : Glutamic acid and glutamine

Brief Description of the Drawings

Fig. 1 shows the amino acid sequence of human IL-2 produced by the present method and having an H-Ala-Pro fragment at its amino terminus (rIL-2). Figures 2 and 3 show the results obtained by FPLC and SDS-PAGE respectively; the analysis procedures are described in Example 1. Figures 4 and 5 show the structural diagrams of plasmid pTF1 and plasmid pTB285 respectively, both of which are described in Reference Example 2 (i).

The present invention is hereinafter more concretely described with Example and Reference Examples, but should not be limited to these.

The transformant employed in the Reference Example 2, i.e. Escherichia coli N4830/pTB285, was deposited on April 25, 1985 under the accession number of IFO 14437 at the Institute for Fermentation, Osaka, (IFO), Japan, and also was deposited on April 30, 1985 under the accession number of FERM P-8199 at the Fermentation Research Institute, Agency of Industrial Science and Technology, Ministry of International Trade and Industry (FRI), Japan and the deposit was converted to a deposit under the Budapest Treaty and the transformant has been stored under the accession number of FERM BP-852 at the institute (FRI).

The transformant Escherichia coli K12 χ 1776/pHGH107 (ATCC 31538) employed in Reference Example 4 is listed in American Type Culture Collection Catalogue of Bacteria, Pharges and rDNA Vectors, sixteenth edition, 1985, and the transformant was deposited on April 23, 1986 at the IFO under the accession number of IFO 14505.

The bioactivity of interleukin-2 was measured in accordance with the method of Hinuma et al. in which interleuking-2-dependent cells are used - [Biochemical Biophysical Research Communications, 109, 363 (1982)].

In the following Examples and Reference Examples, human IL-2 produced by gene engineering techniques and having an H-Met-Ala-Pro fragment at its amino-terminus (polypeptide possessing an amino acid sequence as shown in Fig. 1 and having methionine at its amino terminus) is abbreviated to Met-rIL-2, and that having an H-Ala-Pro fragment at its amino-terminus (polypeptide represented by the amino acid sequence shown in Fig. 1) to rIL-2.

Example 1

Conversion of Met-rIL-2 to rIL-2 with aminopeptidase M:

First, 4.2 mg of Met-rIL-2 obtained in Reference Example 2 (vii) was reacted with 0.17 mg of aminopeptidase M treated with DFP and PCMB - [Journal of Biochemistry, 94 , 619 (1983)] in 4 ml of 0.05M phosphate buffer solution (pH 7.0) at 37°C. Samples were collected from the reaction liquid at 0, 5, 20 and 44 hours after reaction initiation; free amino acids in the reaction liquid were analyzed with a HITACHI 835 model amino acid analyzer (Japan); the behaviour of Met-rIL-2 was analyzed by FPLC. As shown in Table 1, a time dependent increase was observed in the methionine content of the reaction liquid.

Table 1

| Reaction Time (Hours) | Free Amino Acid (Moles/mole Met-rIL-2) |
|---|---|
| 0 | Met (0) |
| 5 | Met (0.13) |
| 20 | Met (0.34) |
| 44 | Met (0.47) |

IN FPLC, both a time-dependent decrease in Peak 2 fraction content, i.e. Met-rIL-2 content, and a time-dependent increase in Peak 1 fraction - (newly appearing) content were observed, as shown in Figure 2. The conversion rate of Peak 2 to Peak 1 at 20 hours after reaction initiation was approx. 40%.

Peak 1 and Peak 2 fractions were then recovered by FPLC using 1.9 ml of a reaction liquid sampled by 20 hours after reaction initiation to prove the newly appearing Peak 1 fraction to be rIL-2 and the Peak 2 fraction to be the remaining Met-rIL-2. Each recovered fraction was then subjected to high performance liquid chromatography - (HPLC), using a tri-fluoroacetic acidacetonitrile system as an elution solvent to remove the polybuffer used in FPLC.

The following conditions were employed:

| | | |
|---|---|---|
| Column | : | Ultrapore RPSC (1.0 x 25 cm, Altex) |
| Column temperature | : | 25°C |
| Elution solvent A | : | 0.1% trifluoroacetic acid-45% acetonitrile |
| Elution solvent B | : | 0.1% trifluoroacetic acid-66% acetonitrile |
| Elution program | : | 0 min. 100% A - 12 min. 100% B |
| Elution rate | : | 3.0 ml/min. |

Each resulting peak fraction of the chromatography was lyophilized, yielding a white powder. Products obtained by FPLC from Peak 1 and Peak 2 fractions were named P1 and P2, respectively. The yield of P1 was 0.28 mg (14%) and that of P2 was 0.72 mg (36%).

Next, P1 and P2 were subjected to protein-chemical analysis. Using 21 µg (1.4 nmole) of P1 and 26 µg (1.7 nmole) of P2 as samples, the amino terminal amino acid sequences of P1 and P2 were determined via automatic Edman degradation using a gas-phase protein sequencer (Applied Biosystems, 470A model). Phenylthiohydantoin amino acids (PTH-amino acids) were identified via HPLC using a Micropac SP-C18 column (Varian). PTH-amino acids detected at each step are shown in Table 2.

Table 2

| | Detected PTH-amino Acids (nmoles) | |
|---|---|---|
| | P1 | P2 |
| 1 | Ala (1.18) | Met (1.30) |
| 2 | Pro (0.71) | Ala (0.73) |
| 3 | Thr (0.47) | Pro (0.53) |
| 4 | Ser (0.23) | Thr (0.22) |
| 5 | Ser (0.12) | Ser (0.08) |

Carboxyl-terminal analysis was conducted as follows. P1 and P2 were introduced into separate glass tubes for hydrazinolysis; anhydrous hydrazine was added. The glass tube was sealed under reduced pressure and heated at 100°C for 6 hours. The resulting hydrazinolysis product, after benzaldehyde treatment, was subjected to free amino acid analysis using a HITACHI 835 model amino acid analyzer. Only threonine was detected in P1 and P2; yields were 51.0% and 48.0% respectively. Based on this fact, the carboxyl-terminal amino acids of P1 and P2 were both identified as threonine. Amino acid composition was analyzed as follows. Each sample was put into a glass tube; constant boiling hydrochloric acid containing 4% thioglycolic acid was added. The glass tube was sealed under reduced pressure and hydrolyzed at 110°C for 24, 48, or 72 hours. Each of the resulting hydrolyzates was analyzed using a HITACHI 835 model amino acid analyzer; after oxidation with performic acid, cystine and cysteine were hydrolyzed in constant boiling hydrochloric acid under reduced pressure for 24 hours and then quantified as cysteic acid, using an amino acid analyzer. Analysis values were calculated by averaging data obtained after hydrolysis for 24, 48 and 72 hours; however, the values for serine and threonine were extrapolated on the assumption that hydrolysis time was 0 hours. The results are shown in Table 3.

Table 3

| | Composition | | | Value estimated on the Basis of cDNA Base Sequence |
|---|---|---|---|---|
| | P1 | P2 | Met-rIL-2 | |
| Asp & Asn | 12.0 | 12.0 | 12.0 | 12 |
| Thr | 12.7 | 12.6 | 12.5 | 13 |
| Ser | 7.6 | 7.7 | 7.7 | 8 |
| Glu & Gln | 18.3 | 18.2 | 18.2 | 18 |
| Pro | 4.7 | 4.7 | 4.7 | 5 |
| Gly | 2.3 | 2.2 | 2.1 | 2 |
| Ala | 5.1 | 5.1 | 5.0 | 5 |
| H-Cys | 2.6 | 2.5 | 2.5 | 3 |
| Val | 4.1 | 4.1 | 3.9 | 4 |
| Met | 3.7 | 4.6 | 4.5 | 4 |
| Ile | 8.1 | 8.1 | 8.0 | 9 |
| Leu | 20.8 | 20.9 | 20.8 | 22 |
| Tyr | 3.1 | 3.2 | 3.0 | 3 |
| Phe | 5.9 | 6.0 | 5.9 | 6 |
| Lys | 10.9 | 11.1 | 11.0 | 11 |
| His | 3.1 | 3.3 | 3.1 | 3 |
| Arg | 4.0 | 4.0 | 3.9 | 4 |
| Trp | 1.1 | 1.3 | 1.2 | 1 |

The homogeneities of P1 and P2 were analyzed by SDS-polyacrylamide gel electrophoresis in accordance with the method of Laemmli [Nature, 222 , 680-685 (1970)]; acrylamide concentration was 15% and sample load was 7 µg for both P1 and P2. As shown in Fig. 3, a single band appeared with both P1 and P2 under both reductive and non-reductive conditions. Based on amino-terminal analysis, amino acid composition analysis and SDS-polyacrylamide gel electrophoresis, it was revealed that P1 and P2 contained rIL-2 and Met-rIL-2 respectively.

The IL-2 activities of P1 and P2 were next determined in accordance with the method of Hinuma et al. [Bilchem. Biophys. Res. Commun., 109, 363 (1982)]. The specific activities of P1 and P2 were 35,900 U/mg and 37,000 U/mg respectively, both nearly equal to the specific activity of Met-rIL-2, 35,800 U/mg, used as starting material in the reaction for conversion of Met-rIL-2 to rIL-2.

The same reaction was conducted using an amount of 5 times of DFP/PCMB-treated aminopeptidase M to Met-rIL-2; the reaction progress was monitored by FPLC and free methionine analysis. Free methionine was detected at ratios of 0.17, 0.29, 0.69, and 0.98 mole/mole Met-rIL-2 at 2, 5, 20 and 44 hours after reaction initiation; rIL-2 peak heights corresponding to the quantities of methionine were observed in FPLC. Thus it can be affirmed that Met-rIL-2 can be converted to rIL-2 almost completely by raising the ratio of enzyme to Met-rIL-2.

Example 2

Conversion of Met-rIL-2 to rIL-2 with aminopeptidase M-Cellulofine:

1.0 ml of 0.05M phosphate buffer solution (pH 7.0) containing 1.05 mg of Met-rIL-2 obtained in Reference Example 2 (vii) was added to 0.5 ml aminopeptidase M-Cellulofine obtained in Reference Example 1 for reaction at 37°C for 4 hours with stirring. After the reaction was completed, the reaction liquid was eluted from the column and recovered fully from the column using 1 ml of 0.05M phosphate buffer solution (pH 7.0). The recovered reaction liquid was subjected to free amino acid analysis and FPLC in accordance with the methods described in Example 1. Free methionine was detected at a ratio of 0.1 mole/mole Met-rIL-2 in amino acid analysis, rIL-2 production being observed in FPLC.

The conversion rate from Met-rIL-2 to rIL-2 was drastically increased by employing a large amount of aminopeptidase M-Cellulofine or a long time reaction. Namely, 10 mg of Met-rIL-2 dissolved in 6 ml of 5 mM ammonium acetate (pH 6.0) were added to 10 ml of aminopeptidase M-Cellulofine. The reaction was carried out at 30°C for 40 hours with stirring. In FPLC the reaction liquid gave a peak at the position of the elution of rIL-2, and the conversion rate was about 50% calculated from the area ratio.

In order to confirm that the peak was really derived from rIL-2, the fraction of this peak was recovered, and polybuffer was removed from the sample by HPLC using Ultrapore RPSC column, and then the fraction was subjected to protein-chemical analysis in accordance with the methods described in Example 1. The aminoterminal amino acid sequence up to five cycles was Ala-Pro-Thr-Ser-Ser-, and amino acid composition was in good agreement with the values of the composition deduced from the cDNA base sequence. The specific activity was 34400 U/mg. These results show that Met-rIL-2 is converted to rIL-2 by reacting Met-rIL-2 with aminopeptidase M-Cellulofine.

Said aminopeptidase M-Cellulofine was able to be used repeatedly, for example, for about 30 times, and in this repeat use the conversion rate was not decreased.

Example 3

Conversion of Met-rIL-2 to rIL-2 with aminopeptidase M-Affi-Gel-15:

To 1.5 ml of aminopeptidase M-Affi-Gel-15 obtained in Reference Example 3 was added 1.0 ml of 0.05 M phosphate buffer solution (pH 7.0) containing 1.05 mg of Met-rIL-2 obtained in Reference Example 2 (vii). The reaction was carried out at 37°C for 4 hours with stirring. The reaction mixture was eluted from the column and reaction liquid was recovered fully from the column using 1 ml of 0.05 M phosphate buffer solution (pH 7.0). The recovered solution was subjected to amino acid analysis and FPLC in accordance with the methods described in Example 1. Free methionine was detected at a rate of 0.05 mole/mole of Met-rIL-2 in amino acid analysis. In FPLC a peak corresponding to rIL-2 was observed.

Example 4

Conversion of Met-rIL-2 to rIL-2 with aminopeptidase M-agarose:

To 1.0 ml of aminopeptidase M-agarose [0.5 U/ml of the gel, manufactured by Pierce Chemical Co. Illinois, U.S.A.] was added 1.0 ml of 0.05 M phosphate buffer solution (pH 7.0) containing 1.05 mg of Met-rIL-2 obtained in Reference Example 2 - (vii). The reaction was carried out at 37°C for 4 hours with stirring. The reaction mixture was eluted from the column and the reaction liquid was recovered fully from the column using 1 ml of 0.05 M phosphate buffer solution (pH 7.0). The recovered solution was subjected to amino acid analysis on free amino acid and to FPLC in accordance with the methods described in Example 1. Free methionine was detected at a rate of 0.05 mole/mole of Met-rIL-2 in amino acid analysis. In FPLC a peak corresponding to rIL-2 was observed.

Example 5

Conversion of Met-rhGH to rhGH with aminopeptidase M:

1.0 mg of Met-rhGH obtained in Reference Example 4 was mixed with 38 µg of aminopeptidase M treated with DFP and PCMB in 1.0 ml of 0.05 M phosphate buffer solution (pH 7.0), and the reaction was carried out at 37°C. 0.2 ml each of samples were collected from the reaction liquid after 0, 5, 24 and 48 hours; free amino acids in the reaction liquid were analyzed in the manner of Example 1. As shown in Table 4, a time-dependent increase was observed in the methionine content of the reaction liquid. rhGH: recombinant human growth hormone

Table 4

| Reaction Time (Hours) | Free Amino Acid (Moles/mole of Met-rhGH) |
|---|---|
| 0 | Met (0) |
| 5 | Met (0.04) |
| 24 | Met (0.24) |
| 44 | Met (0.31) |

Free amino acids other than methionine were not observed. The reaction mixture of 44 hours reaction was applied to FPLC equipped with Mono P column (Pharmacia). In addition to Met-rhGH peak, a peak assumed to be rhGH was observed. This peak fraction was pooled and subjected to protein-chemical analysis in accordance with the method described in Example 1. The amino terminal amino acid sequence was determined to be Phe-Pro-, and the carboxy terminal amino acid was determined to be Phe. The amino acid composition was in good agreement with the values of the amino acid composition deduced from the cDNA base sequence of hGH.

This result shows that Met-rhGH is converted to rhGH by cleaving the amino terminal methionine alone in the reaction of Met-rhGH with aminopeptidase M treated with DFP and PCMB.

Example 6

Conversion of Met-rhGH to rhGH with aminopeptidase M-Cellulofine

1.1 mg of Met-rhGH obtained in Reference Example 4, dissolved in 2 ml of 0.01 M phosphate buffer solution (pH 7.0) were added to 3 ml of aminopeptidase M-Cellulofine. The reaction was carried out at 30°C with stirring. 0.2 ml each of samples were collected from the reaction liquid after 0, 24, and 48 hours; free amino acids in the reaction liquid were analyzed by amino acid analizer. The results showed that free methionine was detected at a rate of 9% and 12% after 24 and 48 hours respectively, and free amino acids other than methionine were not observed. The above facts prove that Met-rhGH is converted to rhGH by cleaving the amino terminal methionine alone in the reaction of Met-rhGh with aminopeptidase M-Cellulofine.

Reference Example 1

Production Method for Immobilized Aminopeptidase M:

0.51 mg of aminopeptidase M was dissolved in 1 ml of 0.2M phosphate buffer solution, pH 7.0 and 1 ml of Formyl Cellulofine (Seikagaku Kogyo, Japan) (a cellulose derivative), previously rinsed with distilled water and a 0.2M phosphate buffer solution (pH 7.0) on a glass filter, was added. The resulting solution was stirred at room temperature for 30 minutes. Next, 20 mg of sodium cyanoborohydride was added; reaction was carried out at 4°C for 20 hours with stirring. After the reaction, the gel was subjected to glass filtration and washed with 0.1M phosphate buffer solution, pH 7.0. The gel was then put into 1 ml of 0.05M monoethanolamine•hydrochloride solution, pH 8.0, and reacted at 4°C for 3 hours, when it was again subjected to glass filtration and washed with a 0.1M phosphate buffer solution, pH 7.0, yielding 0.6 ml of aminopeptidase M-Cellulofine. Protein content of the filtrate and the washings, after dialysis, were determined using a protein assay kit available from BIO-RAD Laboratories; bovine serum albumin was used as the standard protein. The binding ratio of aminopeptidase M to Formyl Cellulofine was found to be 65%; the quantity of aminopeptidase M bound with 1 ml of gel was proved to be 0.55 mg. Using the hydrolysis activity of alanyl-p-nitroanilide as a standard, the enzyme activity of aminopeptidase M-Cellulofine was measured in accordance with a method described in Methods in Enzymology, 19, 514 (1971); the figure proved to be 0.59U per 1 ml gel, 1U representing the quantity of enzyme by which 1 µmole of p-nitroaniline is freed per minute at 37°C and pH 7.0.

Reference Example 2

Production of Non-gylcosylated Human Interleukin-2:

(i) Construction of expression plasmid:

A 1294bp DNA fragment was cleaved from a human IL-2 gene-containing plasmid pILOT 135-8 - [Refer to Example 1 (vii) of the specification of Japanese Patent Application No. 225079/1983 (filed on November 28, 1983) which corresponds to European Patent Application Publication No. 145,390] with restriction enzyme HgiAI. The cleaved 1294bp DNA fragment was treated with T4 DNA polymerase to have flat ends and was linked with an EcoRI linker pTGCCATGAATTCATGGCA using T4 DNA ligase. The resulting DNA was digested with EcoRI, yielding a DNA fragment having translation initiation codon ATG and a human IL-2 gene.

Using T4 DNA ligase, the resulting DNA fragment was inserted into plasmid ptrp781 [Nucleic Acids Research, 11, 3077 (1983)], the EcoRI-PstI site of which had been digested. The resulting expression plasmid pTF1 has both a translation initiation codon and a human IL-2 gene downstream from trp promotor (Fig.4).

Using restriction enzyme StuI, a DNA fragment was cleaved from the pTF1 plasmid and then linked with a BamHI linker. The resulting plasmid DNA was treated with restriction enzymes BamHI and EcoRI, followed by insertion into a plasmid pTB281, which has λPL promotor at its EcoRI-Bam HI site. The resulting expression plasmid was named pTB285 (Fig. 5).

(ii) Production of transformant

Using the pTB285 plasmid obtained above, Escherichia coli N4830 was transformed in accordance with the method of Cohen et al. - [Proceedings of the National Academy of Sciences, USA, 69, 2110 (1972)], yielding a transformant having plasmid pTB285; the transformant was named Escherichia coli N4830/pTB285.

(iii) Cultivation of the transformant:

A transformant Escherichia coli N4830/pTB285 (IFO 14437, FERM BP-852) was inoculated into a 250 ml flask containing 50 ml of a liquid medium - (pH 7.0) containing 1% Bacto triptone (Difco Laboratories, USA), 0.5% Bacto-yeast extract (Difco Laboratories, USA), 0.5% sodium chloride and 50 µg/ml ampicillin, and then subjected to rotary shaking cultivation at 37°C over night. The resulting culture liquid was then transferred to a 5 ℓ jar fermentor containing 2.5 ℓ of M9 medium containing 0.5% Casamino acid, 0.5% glucose and 50 µg/ml ampicillin, after which it was subjected to cultivation under aeration and agitation at 35°C for 6 hours and 42°C for 3 hours, yielding 2.5 ℓ of culture liquid which was then centrifuged to harvest bacterial cells. The cells were frozen at -80°C and stored.

(iv) Extraction:

Twenty grams of the frozen cell sample was uniformly suspended in 100 ml of an extraction solvent (pH 7.0) containing 7M guanidine hydrochloride and 0.1M Tris-HCl, and stirred at 4°C for 1 hour. The resulting suspension was then centrifuged at 28,000 x g for 20 minutes, yielding a supernatant.

(v) Partial purification of interleukin-2 protein

The resulting supernatant, after dialyzation against a 0.01M Tris-HCl buffer solution (pH 8.5), was centrifuged at 19,000 x g for 10 minutes, again yielding a supernatant. The resulting supernatant was passed through a 50 ml column packed with DE52 (DEAE cellulose, Wattman, UK) equilibrated with a 0.01M Tris-HCl buffer (pH 8.5) to adsorb protein; IL-2 was then eluted using a NaCl concentration linear gradient (0-0.15M NaCl, 1 ℓ), yielding an active fraction.

(vi) Purification of interleuking-2 protein:

After concentrated to 5 ml using a YM-5 membrane (Amicon, USA), the active fraction obtained above was subjected to gel filtration using a column (500 ml capacity) packed with Sephacryl S-200 (Pharmacia, Sweden) previously equilibrated with a 0.1M Tris-HCl (pH 8.0)-1M NaCl buffer. Forty milliliters of the resulting active fraction was concentrated to 3 ml using a YM-5 membrane. The resulting concentrate was adsorbed in an Ultrapore RPSC column (Altex, USA) and subjected to HPLC using a trifluoroacetic acid-acetonitrile system as eluent. The following conditions were maintained.

| | | |
|---|---|---|
| Column | : | Ultrapore RPSC (4.6 x 75 mm) |
| Column temperature | : | 30°C |
| Elution solvent A | : | 0.1% trifluoroacetic acid-99.9% water |
| Elution solvent B | : | 0.1% trifluoroacetic acetonitrile |
| Elution program | : | 0 min. (68% A + 32% B) - 25 min. (55% A + 45% B) - 35 min. (45% A + 55% B) - 45 min. (30% A + 70% B) - 48 min. (100% B) |
| Flow rate | : | 0.8 ml/min. |
| Detection wavelength | : | 230 nm |

Ten milliliters of an active fraction of approx. 39 min. of retention time, i.e. a mixture of rIL-2 and Met-rIL-2, was collected.

(vii) Separation of rIL-2 and Met-rIL-2 Using SP-5PW Column:

0.5 ml of a 0.005M ammonium acetate buffer containing the mixutre obtained above (pH 5.0, protein concentration 1.03 mg/ml) was adsorbed on an SP-5PW column for HPLC (0.75 x 7.5 cm, Toyo Soda, Japan) equilibrated with a 0.025M phosphate buffer solution (pH 7.4) to elute protein. Column temperature and flow rate were maintained at 35°C and 0.5 ml/min. respectively. A varian 5,500 model liquid chromatograph was used.

Non-glycosylyzed interleukin-2 eluted in the form of two peaks (Peak A and Peak B). Each peak was subjected to amino-terminal analysis; revealing that Peak A and Peak B contains Met-rIL-2 respectively at a purity of over 99.5%.

Peak A fraction was lyophilized, yielding a white powder.

Reference Example 3

Production method for immobilized aminopeptidase M (aminopeptidase M-Affi-Gel) :

1 ml of 0.1 M sodium bicarbonate containing 0.49 mg of aminopeptidase M was added to 1 ml of Affi-Gel-15 (Bio-Rad Labs., U.S.A.) (derivative of agarose), previously rinsed with cold distilled water. The reaction was carried out at 4°C for 5 hours with stirring. After the reaction, the resultant gels were recovered on glass filter and washed with 0.1 M sodium bicarbonate (pH 8.1). Thus obtained gels were poured into 1 ml of 1 M monoethanolamine-hydrochloric acid (pH 8.0), and the mixture was subjected to reaction at 4°C for one hour with stirring. After the reaction, the reaction mixture was filtered on glass filter and the gels obtained was washed with 6 ml of 0.1 M sodium bicarbonate (pH 8.1) to give 1.6 ml of aminopeptidase M-Affi-Gel-15. The binding efficiency of aminopeptidase M to Affie-Gel-15 was found to be 57%. as determined by measuring the absorbance at 280 nm of the filtrate and the washings after adjusting their pH between 1.5 and 2.0. The quantity of aminopeptidase M bound with 1 ml of gel was calculated as 0.17 mg. The enzyme activity of aminopeptidase M-Affi-Gel-15 was measured as 0.42 U/ml of gel, in accordance with a method described in Reference Example 1 based on the hydrolysis activity on alanyl-p-nitroanilide.

Reference Example 4

Production of Met-recombinant human growth hormone:

(i) Producer:

Transformant Escherichia coli K12 x 1776/pHGH107 (ATCC 31538) was employed.

(ii) Cultivation:

Escherichia coli K12 χ 1776/pHGH107 (ATCC 31538, IFO 14505) was inoculated into a 2 ℓ capacity of flask containing 1 liter of a liquid seed medium (pH 7.0) comprising 1% Bacto-tryptone, 0.5% Bacto-yeast, 0.5% sodium chloride, 10 mg/l tetracycline, hydrochloride, 10 mg/l sodium ampicillin, 20 mg/l thymine and 100 mg/l diaminopymeric acid, and then subjected to rotary shaking cultivation at 37°C overnight. The resultant culture liquid was then transferred to a 50 liter jar fermentor containing 20 liter of a liquid production medium (pH 6.8) containing 1.68% sodium hydrogen phosphate, 0.30% potassium dihydrogenphosphate, 0.10% ammonium chloride, 0.05% sodium chloride. 200 mg/l antifoaming agent, 1.00% glucose, 1.00% casamino acid, 246 mg/l magnesium sulfate, 10 mg/l tetracycline•hydrochloride, 10 mg/l sodium ampicillin, 20 mg/l thymine and 100 mg/l diaminopimeric acid, after which it was subjected to cultivation under aeration and agitation at 37°C for 10 hours. Thus obtained culture fluid was subjected to centrifugation to harvest bacterial cells. The cells were frozen at -80°C and stored.

(iii) Purification of Met-recombinant human growth hormone (Met-rhGH):

Purification of Met-recombinant human growth hormone was performed according to the methods described by Kenneth, et al. (Nature, 293, 408-411, 1981) with some modifications. From frozen cells - (wet weight 53 g), 23 mg of recombinant human growth hormone was purified by 7 M guanidine•HCl extraction, dialysis, DEAE-Toyopearl 650S (Toyo-Soda Ind., Japan), column chromatography, Sephacryl S-200 (Pharmacia, Sweden), gel filtration, Sephadex G-25 (Pharmacia) gel filtration steps.

The product thus obtained was subjected to protein-chemical analysis in accordance with the methods described in Example 1. The product was judged to be homogenous on SDS-polyacrylamide gel electrophoresis. The amino acid composition was in good agreement with that of natural type human growth hormone, except for the fact that one more methionine residue in comparison with the natural type human growth hormone was detected. The amino terminal amino acid sequence of the product was determined to be Met-Phe-Pro-, and the carboxyl terminal amino acid was Phe.

These results are in good agreement with those reported in Nature vol. 281, p. 544 (1979) and vol. 293, p. 408 (1981).

From the above facts, it is proved that the product thus obtained is Met-recombinant human growth hormone.

## Claims

1. A method for removal of the N-terminal methionine from a methionylprotein, which comprises reacting a methionylprotein represented by the formula:

H-Met-X-Pro-Y-OH

wherein X is an amino acid other than proline and Y is a peptide chain, with aminopeptidase to give a protein represented by the formula:

H-X-Pro-Y-OH

wherein X and Y have the same meanings as defined above.

2. A method as claimed in Claim 1, wherein the methionylprotein is produced by gene recombination technology.

3. A method as claimed in Claim 1, wherein the amino acid number of the protein is less than about 400.

4. A method as claimed in Claim 1, wherein the amino acid number of the protein is about 20 to 300.

5. A method as claimed in Claim 1, wherein the molecular weight of the protein is about 200 to 50,000.

6. A method as claimed in Claim 1, wherein the molecular weight of the protein is about 200 to 36,000.

7. A method as claimed in Claim 1, wherein the protein is a lymphokine or a hormone.

8. A method as claimed in Claim 7, wherein the lymphokine is interleukin-2.

9. A method as claimed in Claim 7, wherein the hormone is growth hormone.

10. A method as claimed in Claim 1, wherein the aminopeptidase is aminopeptidase M, leucine aminopeptidase, aminopeptidase PO or aminopeptidase P.

11. A method as claimed in Claim 1, wherein the aminopeptidase is aminopeptidase M.

1
Ala Pro Thr Ser Ser Ser Thr Lys Lys Thr Gln Leu Gln

20
Leu Glu His Leu Leu Leu Asp Leu Gln Met Ile Leu Asn

Gly Ile Asn Asn Tyr Lys Asn Pro Lys Leu Thr Arg Met

40
Leu Thr Phe Lys Phe Tyr Met Pro Lys Lys Ala Thr Glu

60
Leu Lys His Leu Gln Cys Leu Glu Glu Glu Leu Lys Pro

Leu Glu Glu Val Leu Asn Leu Ala Gln Ser Lys Asn Phe

80
His Leu Arg Pro Arg Asp Leu Ile Ser Asn Ile Asn Val

100
Ile Val Leu Glu Leu Lys Gly Ser Glu Thr Thr Phe Met

Cys Glu Tyr Ala Asp Glu Thr Ala Thr Ile Val Glu Phe

120
Leu Asn Arg Trp Ile Thr Phe Cys Gln Ser Ile Ile Ser

133
Thr Leu Thr

Fig.1

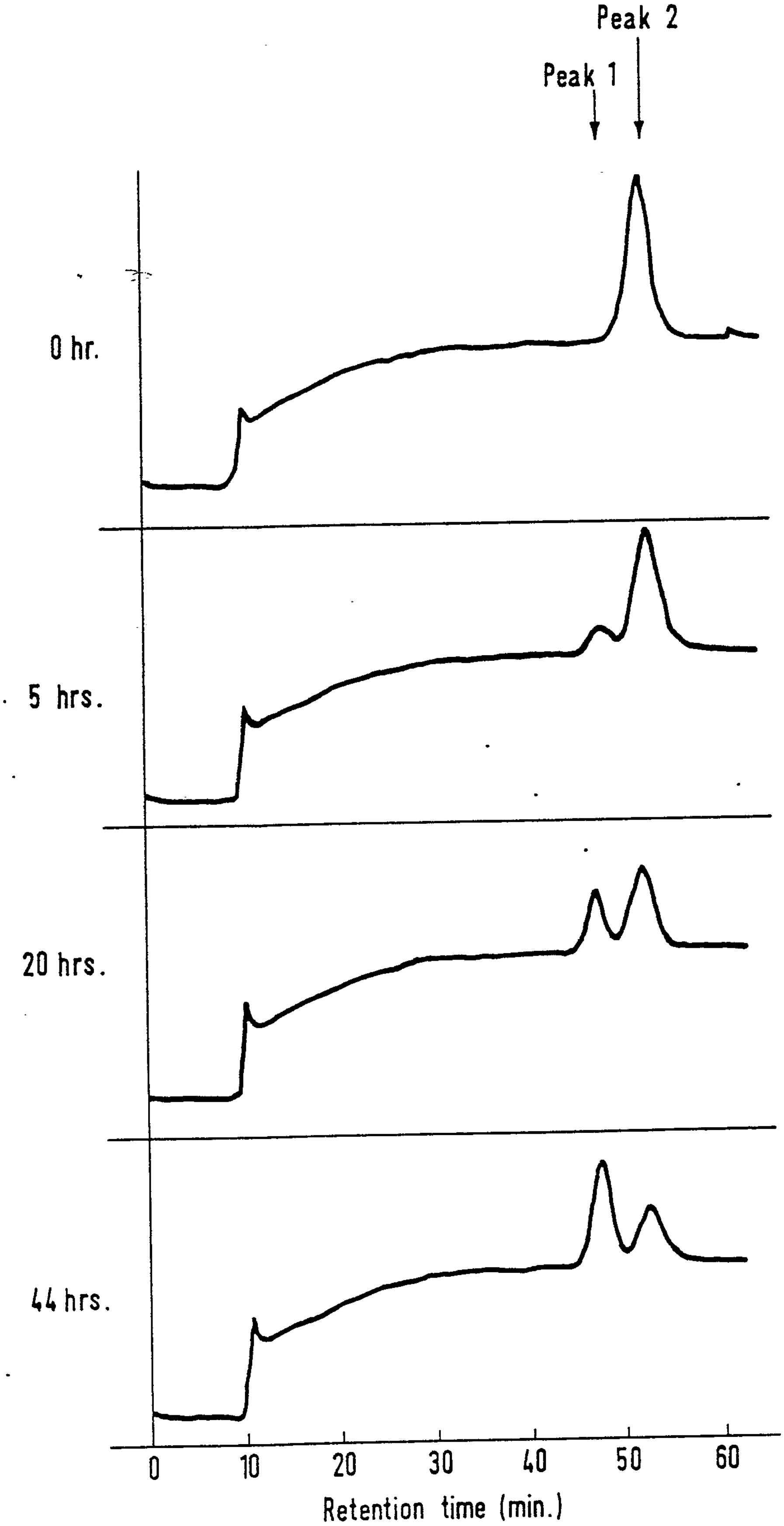

Peak 2
Peak 1
0 hr.
5 hrs.
20 hrs.
44 hrs.
0
10
20
30
40
50
60
Retention time (min.)


Fig.2

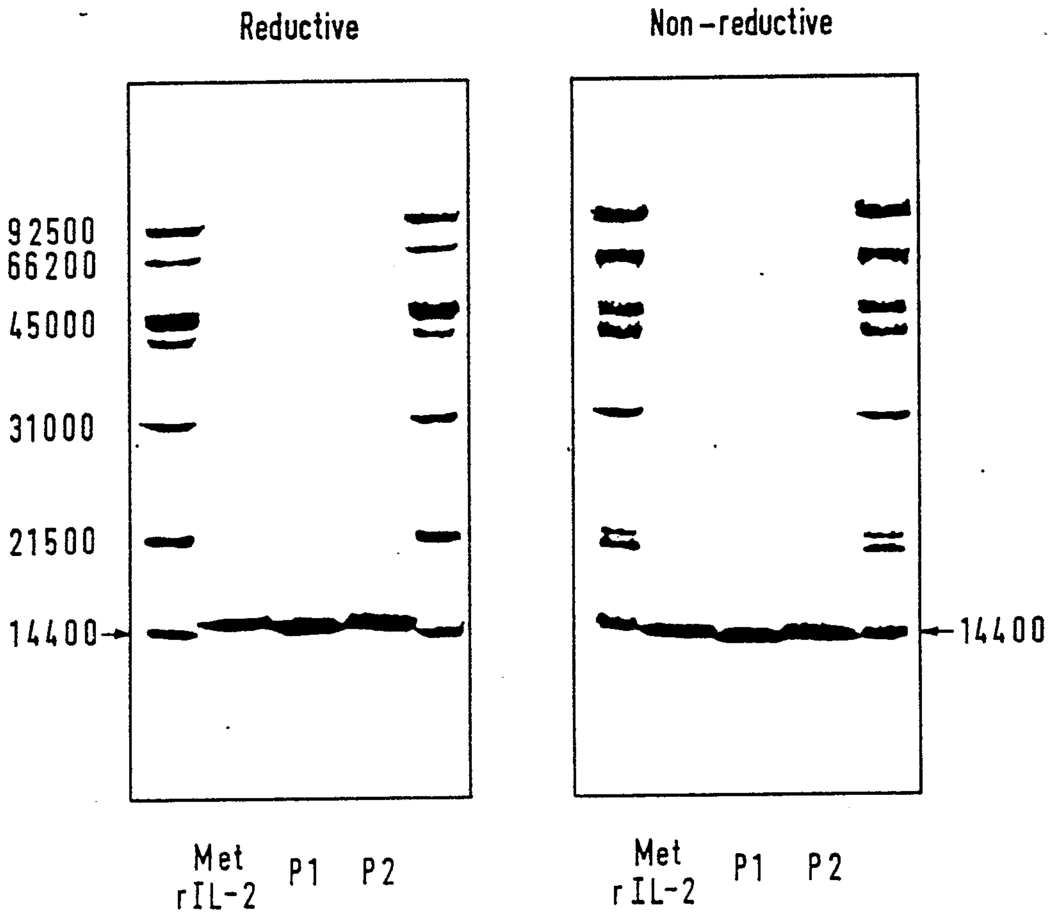
Reductive
Non-reductive
92500
66200
45000
31000
21500
14400
14400
Met
rIL-2
P1
P2
Met
rIL-2
P1
P2

Fig.3

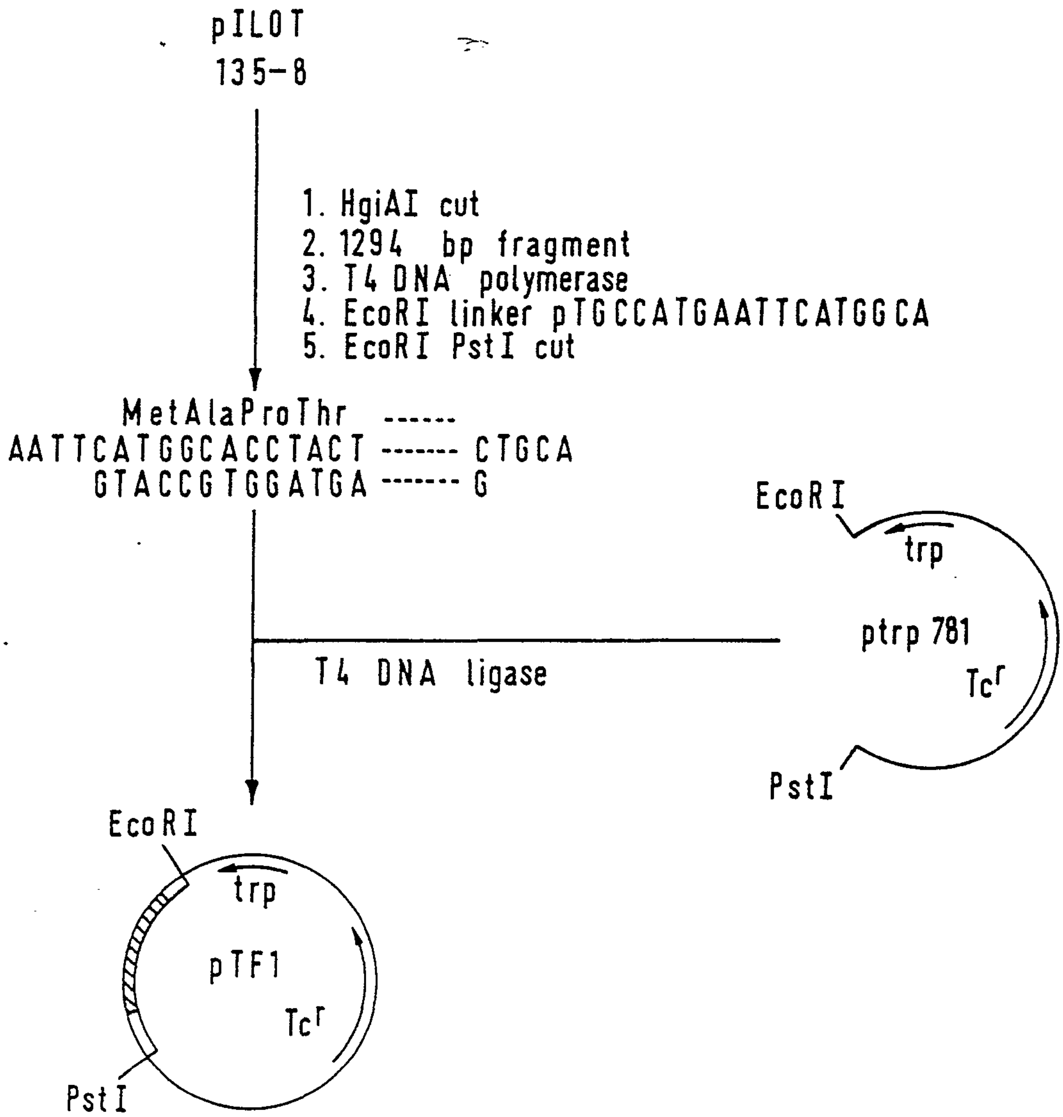
pILOT
135-8
1. HgiAI cut
2. 1294 bp fragment
3. T4 DNA polymerase
4. EcoRI linker pTGCCATGAATTCATGGCA
5. EcoRI PstI cut
MetAlaProThr ------
AATTCATGGCACCTACT ------- CTGCA
GTACCGTGGATGA ------- G
EcoRI
trp
ptrp 781
Tc^r
PstI
T4 DNA ligase
EcoRI
trp
pTF1
Tc^r
PstI

Fig.4

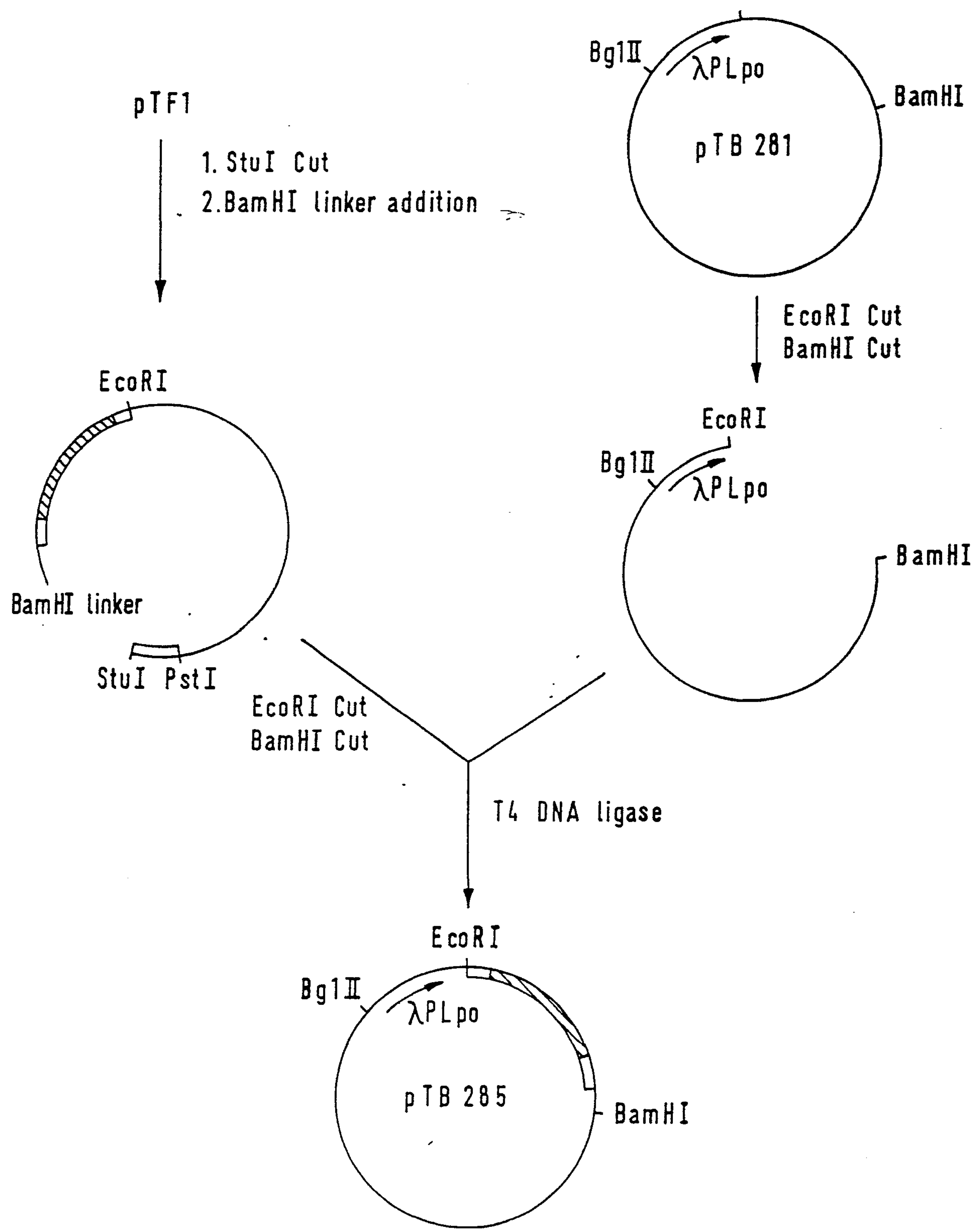
pTF1
1. StuI Cut
2.BamHI linker addition
Bg1II
λPLpo
BamHI
pTB 281
EcoRI Cut
BamHI Cut
EcoRI
BamHI linker
StuI PstI
EcoRI
Bg1II
λPLpo
BamHI
EcoRI Cut
BamHI Cut
T4 DNA ligase
EcoRI
Bg1II
λPLpo
pTB 285
BamHI

Fig.5

European Patent Office

# EUROPEAN SEARCH REPORT

Application number

EP 86304144.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | WO - A1 - 86/01 229 (BIO TECHNOLOGY GENERAL CORP.)<br>* Claims 1,15,16,21-27,30,32,35, 53 * | 1,2,7, 9 | C 12 P 21/00<br>C 12 P 21/02<br>C 12 P 21/06<br>C 12 N 15/00 |
| X | EP - A1 - 0 020 290 (SCHERING AKTIENGESELLSCHAFT BERLIN UND BERG-KAMEN)<br>* Abstract * | 1 | |
| D,A | WO - A1 - 84/02 351 (NORDISK INSULINLABORATORIUM)<br>* Claims 1-3 * | 1,7,9, 10 | |
| D,A | SCIENCE, vol. 198, no. 4321, December 9, 1977, Washington D.C.<br>K. ITAKURA et al. "Expression in Escherichia coli of a Chemically Synthesized Gene for the Hormone Somatostatin"<br>pages 1056-1063<br>* Page 1057; fig. 1 * | 1 | TECHNICAL FIELDS SEARCHED (Int. Cl.4)<br>C 12 P<br>C 12 N |

The present search report has been drawn up for all claims

| Place of search | Date of completion of the search | Examiner |
|---|---|---|
| VIENNA | 08-09-1986 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82